# EUROPEAN PATENT APPLICATION

(11) **EP 4 596 542 A1**
(43) Date of publication of application: **06.08.2025**
(21) Application number: 23873218.4
(22) Date of filing: 27.09.2023
(51) Int. Cl.: C07D 407/04, A61K 31/351, A61K 9/14, A61P 3/10

(54) **NOVEL CO-CRYSTAL OF ENAVOGLIFLOZIN**

(30) Priority: 28.09.2022 KR 20220123680
(71) Applicant: Daewoong Pharmaceutical Co., Ltd., Hwaseong-si, Gyenoggi-do 18623 (KR)
(72) Inventor: YOON, Youn Jung, Yongin-si Gyeonggi-do 16899 (KR); YOON, Hee Kyoon, Cheongju-si Chungcheongbuk-do 28774 (KR); CHOI, Ji Soo, Seoul 06310 (KR); JI, Hye Young, Yongin-si Gyeonggi-do 17010 (KR); LIM, Hyun Woo, Yongin-si Gyeonggi-do 17066 (KR)
(74) Representative: Plasseraud IP
(86) International application number: PCT/KR2023/015051
(87) International publication number: WO 2024/072109

(57) **Abstract**

The present invention relates to a co-crystal of enavogliflozin. The enavogliflozin co-crystal of the present invention enhances the solubility of enavogliflozin in artificial gastric fluid and artificial intestinal fluid. This improved solubility of enavogliflozin may be particularly advantageous for the development of oral formulations for indications requiring high-dose administration.

The enavogliflozin co-crystal also exhibits stability equivalent to that of existing crystalline forms of enavogliflozin, making it highly valuable as an active pharmaceutical ingredient of enavogliflozin. Furthermore, the co-crystal of the present invention has a longer half-life and higher in vivo exposure (AUCinf) compared to the existing crystalline form A, resulting in prolonged efficacy, and thus can be usefully employed in the development of various indications and/or formulations requiring such properties.

## Description

### [Technical Field]

The present invention relates to a co-crystal of enavogliflozin.

### [Background Art]

The selection of preferable solid forms of active ingredients such as pharmaceutically acceptable salts, co-crystals, polymorphs, and pseudo-polymorphs thereof has a very important impact on the development of a manufacturing process for an active pharmaceutical ingredient and the dosage form design and formulation of a drug product.

Specifically, the salts and cocrystals of an active pharmaceutical ingredient, and polymorphs thereof affect the final stage of the manufacture of an active pharmaceutical ingredient, that is, the recrystallization yield, process speed and purity in recrystallization and purification processes. Depending on the size or shape of a crystal, the speed in the crystallization process may vary, which affects the productivity and manufacturing costs.

**In** addition, in pharmaceutical aspects, physicochemical properties such as hygroscopicity, stability, solubility, particle flowability, and a dissolution rate are affected by a salt, a co-crystal, a polymorph, or a pseudo-polymorph thereof or a polymorph thereof and thus become factors that determine the production process, production and storage conditions, and shelf life of a drug product.

Meanwhile, when the bioavailability of a drug is affected by the physical properties of an active pharmaceutical ingredient, more attention is required in the selection of a salt, a co-crystal, a polymorph, or a pseudo-polymorph thereof or a polymorph thereof is very important in terms of technology development and approval of the drug.

A purpose of the present invention is to explore a novel co-crystal of enavogliflozin, develop a co-crystal that can maximize pharmacological activity by analyzing physicochemical properties, and thus improve low solubility, which is the disadvantage of existing polymorphs.

Sodium glucose cotransporter 2 (SGLT-2) is a transporter that, along with sodium glucose cotransporter 1 (SGLT-1), is responsible for excessive blood sugar reabsorption in the kidneys, with SGLT-2 playing the major role. Therefore, when an SGLT-2 inhibitor blocks the SGLT-2 transporter, the amount of blood sugar excreted in the urine increases, which ultimately lowers blood sugar levels and releases calories from the blood sugar, resulting in weight loss.

One of the drugs developed as an SGLT-2 inhibitor that can be useful as a therapeutic agent for type 2 diabetes due to such effects is enavogliflozin represented by Chemical Formula 1 below, which is disclosed in Korean Unexamined Patent Application Publication No. 2014-0022086 (Patent Document 1).

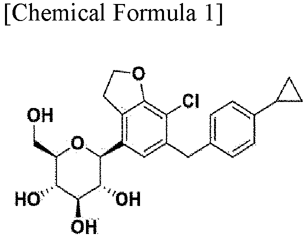

In addition, a crystalline form of enavogliflozin and a method of manufacturing a crystalline form of enavogliflozin have been disclosed in Korean Unexamined Patent Application Publication No. 2017-0142904 (Patent Document 2).

However, the crystalline form of enavogliflozin disclosed in Korean Unexamined Patent Application Publication No. 2017-0142904 (Patent Document 2) has a disadvantage of low solubility of 0.25 mg/mL.

Therefore, the present inventors conducted research on a co-crystal that can improve the solubility of enavogliflozin and confirmed that a specific co-crystal form also has excellent stability during preparation and is able to improve solubility, thereby completing the present invention.

### [Related Art Documents]

### [Patent Documents]

Patent Document 1. Korean Unexamined Patent Application Publication No. 2014-0022086
Patent Document 2. Korean Unexamined Patent Application Publication No. 2017-0142904

### [Disclosure]

### [Technical Problem]

The present invention is directed to providing a novel co-crystal of enavogliflozin with excellent stability and excellent solubility and a preparation method thereof. The present invention is also directed to providing a pharmaceutical composition, which includes the novel co-crystal of enavogliflozin as an active ingredient.

### [Technical Solution]

One aspect of the present invention provides a novel co-crystal of enavogliflozin represented by Chemical Formula 2 below and a preparation method thereof.

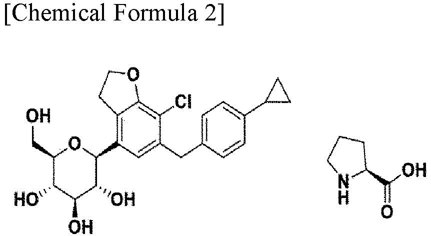

The present inventors attempted to design a co-crystal by selecting amino acids and organic acids that have high solubility and are rich in NH, N, O, and OH to overcome the low solubility of enavogliflozin.

The amino acids and organic acids selected to prepare a co-crystal include aspartic acid, acetylsalicylic acid, citric acid, nicotinic acid, nicotinamide, beta-cyclodextrin, propylene glycol, phenylalanine, lactose monohydrate, L-proline, urea, L-lysine, L-pyroglutamic acid, orotic acid monohydrate, maleic acid, malic acid, L-ascorbic acid, fumaric acid, succinic acid, malonic acid, oxalic acid trihydrate, L-tartarate, sodium chloride, L-serine, L-arginine, L-valine, L-methionine, threonine, glycine, L-alanine, L-cysteine, L-tryptophan, L-asparagine, L-isoleucine, L-glutamine, L-histidine, and L-glutamic acid.

The preparation of co-crystals of enavogliflozin was attempted using the above-mentioned 38 types of amino acids and organic acids, ensuring three types of co-crystals such as an enavogliflozin/proline co-crystal, an enavogliflozin/methionine co-crystal, and an enavogliflozin/proline hydrate co-crystal.

As a result of evaluating solubility in pH 1.2 and pH 6.8 eluates for the enavogliflozin/proline co-crystal, the enavogliflozin/methionine co-crystal, and the enavogliflozin/proline hydrate co-crystal, the three types of co-crystals, that is, the enavogliflozin/proline co-crystal, the enavogliflozin/methionine co-crystal, and the enavogliflozin/proline hydrate co-crystal, showed higher solubility than the crystalline form of enavogliflozin for up to the first 2 hours, confirming that the initial solubility was improved through the co-crystals.

However, the enavogliflozin/proline hydrate co-crystal had a problem of low-reproducibility to prepare a co-crystal during scale-up, and the enavogliflozin/methionine co-crystal was expected to lower stability due to a low melting point of 106 °C. As a result of a 4-week stress stability test at 60 °C, it was confirmed that the purity of enavogliflozin/methionine co-crystal decreased by approximately 3% in only 4 weeks, showing lower stability than the crystalline form of enavogliflozin and the enavogliflozin/proline co-crystal. Accordingly, it was confirmed that the enavogliflozin/proline co-crystal had the best stability and solubility.

For experimental optimization, a method of reproducibly preparing the enavogliflozin/proline co-crystal was established, and the solubility of the enavogliflozin/proline co-crystal prepared by this method was increased by approximately 21% in an artificial gastric fluid, and increased by approximately 2-fold or more in artificial intestinal fluid compared to the existing crystalline forms of enavogliflozin, and the initial solubility was also increased. In addition, the improved effect was confirmed in an oral absorption effect and actual mouse pharmacokinetic experiment due to the above solubility characteristic.

Accordingly, using the enavogliflozin/proline co-crystal of the present invention, it is possible to manufacture an oral preparation with improved solubility of enavogliflozin. The improved solubility of enavogliflozin may be advantageous for the development of oral preparations for indications requiring high-dose administration.

The enavogliflozin/proline co-crystal is formed by the hydrogen bond between one molecule of enavogliflozin and one molecule of proline (L-proline) at a ratio of 1:1.

Since a co-crystal is formed by the hydrogen bond between proline with high solubility in water and enavogliflozin, enavogliflozin is also dissolved in water when proline dissolves due to the interaction with the proline, and therefore the solubility in water and the solubility in artificial gastric fluid and artificial intestinal fluid increase.

The enavogliflozin/proline co-crystal of the present invention is a novel solid form of enavogliflozin, which has not been reported.

In one embodiment of the present invention, an enavogliflozin/proline co-crystal, which is characterized by an X-ray powder diffraction pattern having four or more, e.g., 4, 5, 6, 7, 8 or more, diffraction peaks at 2[θ] values selected from 4.72 ± 0.2, 6.81 ± 0.2, 7.93 ± 0.2, 8.59 ± 0.2, 14.75 ± 0.2, 15.21 ± 0.2, 17.23 ± 0.2, 18.80 ± 0.2, 21.19 ± 0.2, 24.42 ± 0.2, and 27.29 ± 0.2 in powder X-ray diffraction (PXRD) analysis is provided.

Particularly, the X-ray powder diffraction pattern has a diffraction peak at 2[θ] values selected from 6.81 ± 0.2, 8.59 ± 0.2, 14.75 ± 0.2, 17.23 ± 0.2, and 18.80 ± 0.2.

More specifically, the enavogliflozin/proline co-crystal is characterized by an X-ray powder diffraction pattern with peak positions matching those listed in Table 1 below.

**[Table 1]**

| 2θ(±0.2°) | d (Å) | I/Iₒ (%) |
|---|---|---|
| 4.72 | 18.7 | 33.3 |
| 6.81 | 13.0 | 100.0 |
| 7.93 | 11.1 | 47.0 |
| 8.59 | 10.3 | 63.1 |
| 14.75 | 6.0 | 81.6 |
| 15.21 | 5.8 | 31.0 |
| 17.23 | 5.1 | 50.9 |
| 18.80 | 4.7 | 63.2 |
| 21.19 | 4.2 | 22.5 |
| 24.42 | 3.6 | 28.3 |
| 27.29 | 3.3 | 25.4 |

In another embodiment of the present invention, the enavogliflozin/proline co-crystal shows an endothermic peak with an endothermic onset temperature of 217.71 °C ± 3 °C and an endothermic peak temperature of 219.42 °C ± 3 °C in differential scanning calorimetry (DSC). The enavogliflozin/proline co-crystal according to the present invention is in a form where 1 equivalent of proline is bound to 1 equivalent of enavogliflozin. According to nuclear magnetic resonance (NMR) analysis, it can be confirmed that the enavogliflozin/proline co-crystal is composed of one molecule of enavogliflozin and one molecule of proline in the H-NMR spectrum.

Another aspect of the present invention provides a method of preparing the enavogliflozin/proline co-crystal.

Although not limited thereto, the enavogliflozin/proline co-crystal is prepared by a process which includes (a) mixing enavogliflozin with an organic solvent and adding proline to the resulting mixture; (b) stirring the resulting product of step (a); and (c) vacuum-drying the resulting product of step (b) to obtain enavogliflozin/proline co-crystal.

The method of the present invention for preparing an enavogliflozin/proline co-crystal will be described step by step in detail below.

### (a) Mixing enavogliflozin and organic solvent and adding proline

First, the method of the present invention includes mixing solid enavogliflozin and an organic solvent and adding proline to the resulting mixture. The physicochemical form of the solid enavogliflozin used herein is not particularly limited. For example, the solid may be crystalline form A, crystalline form B, crystalline form C or crystalline form D of enavogliflozin, or amorphous enavogliflozin, reported to have the following X-ray diffraction spectrum, according to Experimental Example 4 in Korean Unexamined Patent Application Publication No. 2017-0142904.
Crystalline form A: the crystalline form with an X-ray diffraction (XRD) spectrum having peaks at 2[θ] values selected from 6.2° ± 0.2°, 7.2° ± 0.2°, 8.8° ± 0.2°, 17.6° ± 0.2°, 19.0° ± 0.2°, 22.5° ± 0.2°, and 25.1° ± 0.2°
Crystalline form B: the crystalline form with an X-ray diffraction (XRD) spectrum having peaks at 2[θ] values selected from 7.0° ± 0.2°, 14.9° ± 0.2°, 17.7° ± 0.2°, 18.8° ± 0.2°, 20.6° ± 0.2°, 21.8° ± 0.2°, and 23.5° ± 0.2°
Crystalline form C: the crystalline form with an X-ray diffraction (XRD) spectrum having peaks at 2[θ] values selected from 5.6° ± 0.2°, 7.3° ± 0.2°, 15.7° ± 0.2°, 17.2° ± 0.2°, 18.9° ± 0.2°, 21.2° ± 0.2°, and 21.9° ± 0.2°
Crystalline form D: the crystalline form with an X-ray diffraction (XRD) spectrum having peaks at 2[θ] values selected from 5.5° ± 0.2°, 7.2° ± 0.2°, 15.3° ± 0.2°, 17.2° ± 0.2°, 17.6° ± 0.2°, 18.9° ± 0.2°, and 21.1° ± 0.2°
Each of the crystalline forms A, B, C, and D is specified by the X-ray diffraction spectra having four or more, e.g., 4, 5, 6, 7, 8 or more, peaks at the presented 2[θ] values.

The organic solvent of step (a) may dissolve enavogliflozin and proline well, and any organic solvent which can produce an enavogliflozin/proline co-crystal can be used. An organic solvent that has been proven as a solvent effective in producing an enavogliflozin/proline co-crystal in high yield and removing excess proline may be one or more types of organic solvents selected from the group consisting of methanol, ethanol, isopropyl alcohol, 1-butanol, acetone, tetrahydrofuran, acetonitrile, ethyl acetate, dichloromethane, methyl t-butyl ether (MTBE), toluene, and dioxane. The organic solvent may be a single solvent or mixed solvent.

Preferably, the organic solvent is one or more types of selected from the group consisting of methanol, ethanol, isopropyl alcohol, and acetone. More preferably, the organic solvent is ethanol.

The organic solvent of step (a) may be used in a volume 10 times or more the weight of enavogliflozin, such as 10 to 200 times, for example, 10, 15, 20, 25, 30, 35, 40, 45, 50, 60, 70 80, 90, 100, 120, 150, or 200 times the weight of enavogliflozin.

The amount of an organic solvent used may vary depending on the type of the organic solvent or whether it is used as a single solvent or a mixed solvent. For example, in the case of methanol, dioxane, or tetrahydrofuran, co-crystals may be produced with the solvent only 10 times the weight of enavogliflozin. In the case of ethanol, isopropyl alcohol, 1-butanol, acetone, or acetonitrile, co-crystals may be produced with the solvent in an amount 50 times the weight of enavogliflozin. When ethyl acetate is used as a solvent, due to the low solubility of enavogliflozin, crystallization is required after heating at a level 50 times the weight of enavogliflozin. Dichloromethane does not dissolve enavogliflozin well, and therefore is required in an amount 100 times the weight of enavogliflozin. MTBE and toluene dissolve enavogliflozin very poorly and therefore are required in amounts 150 times or more the weight of enavogliflozin.

Accordingly, by considering the solubility of enavogliflozin in a solvent and the degree of co-crystal production, the type and mixing of organic solvents may be selected and the amount of organic solvent used may be adjusted. If necessary, heating may be applied concurrently to dissolve enavogliflozin.

In still another embodiment of the present invention, the organic solvent may be ethanol, and may be used in a volume 10 to 60 times the weight of enavogliflozin.

In yet another embodiment of the present invention, proline is added in a molar ratio of 1 to 1.5 equivalents with respect to enavogliflozin.

### (b) Stirring the resulting product

Next, the method of the present invention includes stirring the resulting product of step (a).

In yet another embodiment of the present invention, the stirring of step (b) may be performed at 0 to 50 °C, for example, 15 to 40 °C, preferably, 15 to 30 °C, and more preferably, 20 to 25 °C.

In yet another embodiment of the present invention, the stirring of step (b) may be performed for 0.5 to 24 hours, preferably, 1 to 12 hours, and more preferably, 4 to 8 hours.

### (c) Vacuum drying the resulting product and obtaining enavogliflozin/proline co-crystal

Finally, the method of the present invention includes vacuum-drying the resulting product of step (b) and obtaining an enavogliflozin/proline co-crystal.

In yet another embodiment of the present invention, the vacuum drying of step (c) may be performed at 30 to 65 °C, preferably, 40 to 55 °C, and more preferably, 45 to 50 °C for 8 to 12 hours.

In this way, as an SGLT-2 inhibitor, an enavogliflozin/proline co-crystal having 1 equiv. of proline binding to 1 equiv. of enavogliflozin, which can be used as a therapeutic agent for type 2 diabetes, regulating blood sugar by excreting the blood sugar through urine by suppressing resorption of the blood sugar in the kidneys, may be prepared.

The present invention also provides a pharmaceutical composition which includes the enavogliflozin/proline co-crystal as an active ingredient, and a pharmaceutically acceptable carrier.

The pharmaceutical composition may be for treating or preventing diabetes, but the present invention is not limited thereto.

The enavogliflozin/proline co-crystal according to the present invention may be administered in a variety of oral and parenteral dosage forms in clinical administration, and is prepared using a diluent or excipient such as a filler, an extender, a binder, a wetting agent, a disintegrant, or a surfactant, which is normally used in formulation.

### [Advantageous Effects]

The enavogliflozin/proline co-crystal of the present invention enhances the solubility of enavogliflozin in artificial gastric fluid and artificial intestinal fluid. This improved solubility of enavogliflozin may be particularly advantageous for the development of oral formulations for indications requiring high-dose administration. Moreover, the stability of the enavogliflozin/proline co-crystal is equivalent to that of existing crystalline forms of enavogliflozin, rendering it highly valuable as an active pharmaceutical ingredient of enavogliflozin. In addition, the enavogliflozin/proline co-crystal of the present invention exhibits a longer half-life and a higher exposure in the body (AUC_{inf}) than the existing crystalline form A, which allows for longer lasting efficacy, and therefore can be useful in the development of various indications and/or formulations requiring them.

### [Description of Drawings]

FIG. 1 shows the result of H-nuclear magnetic resonance (NMR) spectra for an enavogliflozin/proline co-crystal prepared in Preparation Example 2.
FIG. 2 shows the result of H-NMR spectra for crystalline form A of enavogliflozin prepared according to Preparation Example 1.
FIG. 3 shows the result of powder X-ray diffraction (PXRD) pattern for the enavogliflozin/proline co-crystal prepared according to Preparation Example 2.
FIG. 4 shows the result of PXRD pattern for the crystalline form A of enavogliflozin prepared in Preparation Example 1.
FIG. 5 shows the results of a differential scanning calorimetry (DSC) curve for the enavogliflozin/proline co-crystal prepared according to Preparation Example 2.
FIG. 6 shows the results of DSC curves for the enavogliflozin/proline co-crystal, proline, and crystalline forms of enavogliflozin, prepared according to examples of the present invention.
FIG. 7 shows the comparison in the solubility of the enavogliflozin/proline co-crystals and crystalline form of enavogliflozin prepared according to examples of the present invention in artificial intestinal fluid.

### [Modes of the Invention]

Hereinafter, the advantages and features of the present invention and the methods of accomplishing the same may be clearly understood with reference to the detailed description of exemplary embodiments and the accompanying drawings. However, the present invention is not limited to the exemplary embodiments disclosed below, and may be embodied in many different forms. These exemplary embodiments are merely provided to complete the disclosure of the present invention and fully convey the scope of the present invention to those of ordinary skill in the art, and the present invention should be defined by only the accompanying claims.

### [Preparation Example 1] Preparation of crystalline form A of enavogliflozin

The crystalline form A of enavogliflozin reported from Experimental Example 4 in Korean Unexamined Patent Application Publication No. 2017-0142904 was prepared.

Ethyl acetate (15 times the weight of crude enavogliflozin) was added to crude enavogliflozin, stirred under reflux to dissolve, and then cooled to room temperature. A suspension was formed at room temperature, and additionally stirred for 30 minutes. Isopropyl ether (15 times the weight of crude enavogliflozin) was added dropwise to the resulting mixture over 30 minutes, and further stirred at room temperature for 30 minutes. The resulting precipitate was filtered, washed with ethyl acetate (twice the weight of crude enavogliflozin) at 0 °C and dried in a vacuum oven (50 °C, 12 hrs), thereby obtaining white crystals (yield: 88.3%).

### [Preparation Example 2] Preparation of enavogliflozin/proline co-crystal

300 mg of crystalline form A of enavogliflozin and 10 mL of ethanol were added and then stirred at room temperature for 20 minutes. Afterward, 77.3 mg (1 equiv.) of L-proline was added thereto and stirred at 20 to 25 °C for 1 hour. The precipitated crystals were filtered under reduced pressure, washed with 1 mL of ethanol, and dried under vacuum at 50 °C for 12 hours, thereby obtaining a crystalline form of enavogliflozin/proline co-crystal with a yield of 78%.

### [Preparation Example 3] Evaluation of 13 types of solvents for forming L-proline co-crystal

100 mg (0.224 mmol, 1 equiv.) of crystalline form A of enavogliflozin was dissolved in each solvent, L-proline (0.224 mmol, 1 equiv.) was added at room temperature, and the resulting solution was stirred for 1 hour or more, after which the formation of a proline co-crystal was evaluated.

**[Table 2]**

| **Solvent** | **Amount of solvent used** | **Whether co-crystal is produced** |
|---|---|---|
| Methanol | 10 mL | O |
| Ethanol | 5 mL | O |
| Isopropyl alcohol | 5 mL | O |
| 1-Butanol | 5 mL | O |
| Acetone | 5 mL | O |
| Tetrahydrofuran | 1 mL | O |
| Acetonitrile | 5 mL | O |
| Ethyl acetate | 15 mL or more | O |
| Dichloromethane | 10 mL or more | O |
| Methyl t-butyl ether (MTBE) | 15 mL | O |
| Toluene | 15 mL | O |
| Dioxane | 1 mL | O |
| DMSO | 0.1 mL | X |

In the case of methanol, dioxane, and tetrahydrofuran, enavogliflozin was completely dissolved even with a solvent volume in an amount 10 times the weight of enavogliflozin, and upon addition of L-proline, it was precipitated as co-crystals. In the case of ethanol, isopropyl alcohol, 1-butanol, acetone, and acetonitrile, co-crystals were produced even with a solvent in an amount 50 times the weight of enavogliflozin.

It can be confirmed that large amounts of ethyl acetate and dichloromethane had to be used to dissolve enavogliflozin, and when L-proline was added, it was precipitated as co-crystals. When ethyl acetate was used as a solvent, due to the low solubility of enavogliflozin, crystallization is required after heating at a level 50 times the weight of enavogliflozin. Dichloromethane also dissolves enavogliflozin poorly, confirming that it is needed in an amount 100 times the weight of enavogliflozin is required.

MTBE or toluene should have been used in an amount 150 times or more the weight of enavogliflozin due to the very low solubility of enavogliflozin in these solvents, but when L-proline was added, it can be confirmed that co-crystals were gradually produced.

In contrast, it was confirmed that DMSO easily dissolves co-crystals, and therefore no precipitation was observed.

The amount of solvent needed to be adjusted depending on the solubility according to the polarity of the solvent. However, in the case of solvents affecting the solubility of enavogliflozin, L-proline, and co-crystals, proline co-crystals could not be obtained, but when a certain level of solubility was observed, the proline co-crystals could be secured.

### [Experimental Example 1] NMR spectrum

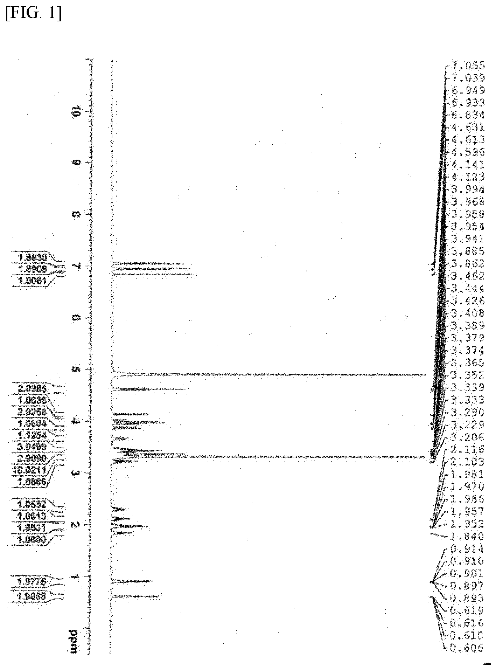

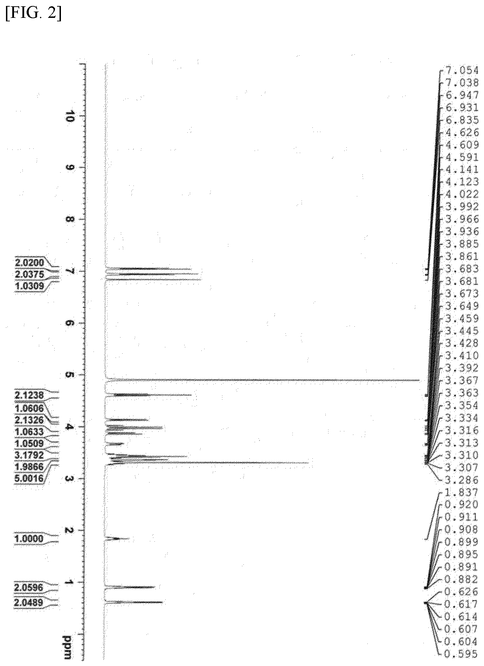

An H-NMR spectrum was detected for a crystalline form of enavogliflozin obtained according to Preparation Example 2. As a result, the result of the H-NMR spectra for the enavogliflozin/proline co-crystal as shown in FIG. 1 could be obtained. FIG. 2 shows the result of the H-NMR spectra for the crystalline form A of enavogliflozin in Preparation Example 1. According to FIG. 1, it can be confirmed that the stoichiometric ratio of enavogliflozin/proline is exactly 1:1, and the peak is integrated to show that the crystalline form of enavogliflozin obtained according to Preparation Example 2 is an enavogliflozin/proline co-crystal.

### [Experimental Example 2] Powder X-ray diffraction (PXRD)

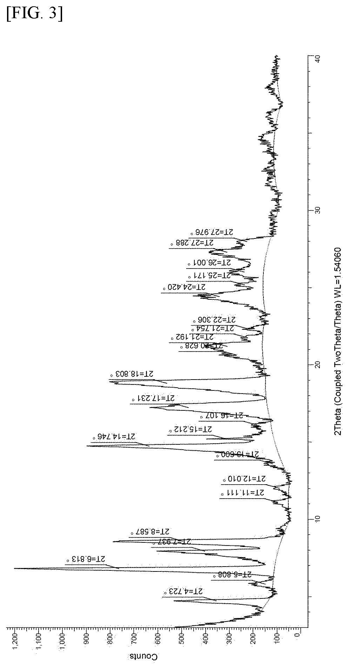

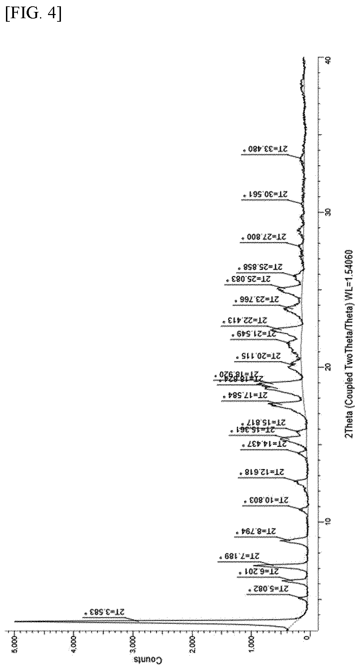

PXRD was performed using Bruker PXRD (30 kV, 10 mA, Cu target) on the enavogliflozin/proline co-crystal obtained according to Preparation Example 2. 2θ scanning was performed at 5 to 40° with 0.02° step size, obtaining the result in FIG. 3. The PXRD results shown in FIG. 3 were clearly distinguished from the PXRD results for the crystalline form A of enavogliflozin in Preparation Example 1 (FIG. 4).

**[Table 3]**

| 2θ(±0.2°) | d (Å) | I/Iₒ (%) | 2θ(±0.2°) | d (Å) | I/Iₒ (%) |
|---|---|---|---|---|---|
| 4.72 | 18.7 | 33.3 | 17.23 | 5.1 | 50.9 |
| 5.81 | 15.2 | 4.9 | 18.80 | 4.7 | 63.2 |
| 6.81 | 13.0 | 100.0 | 20.63 | 4.3 | 16.7 |
| 7.93 | 11.1 | 47.0 | 21.19 | 4.2 | 22.5 |
| 8.59 | 10.3 | 63.1 | 21.75 | 4.1 | 9.5 |
| 11.11 | 8.0 | 6.8 | 22.31 | 4.0 | 8.9 |
| 12.01 | 7.4 | 6.0 | 24.42 | 3.6 | 28.3 |
| 13.60 | 6.5 | 6.2 | 25.17 | 3.5 | 11.7 |
| 14.75 | 6.0 | 81.6 | 26.00 | 3.4 | 12.3 |
| 15.21 | 5.8 | 31.0 | 27.29 | 3.3 | 25.4 |
| 16.11 | 5.5 | 9.0 | 27.98 | 3.2 | 14.5 |

### [Experimental Example 3] Differential scanning calorimetry (DSC)

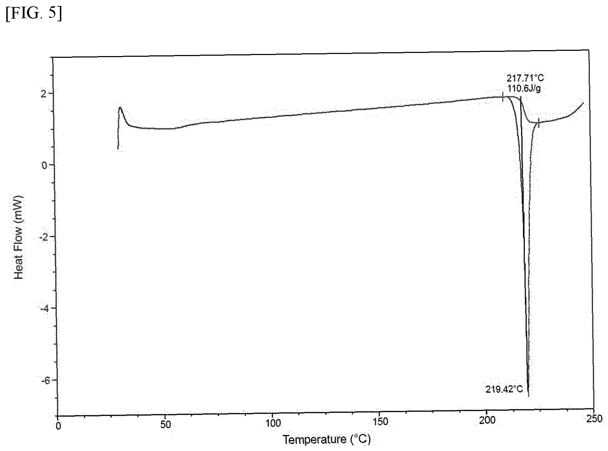

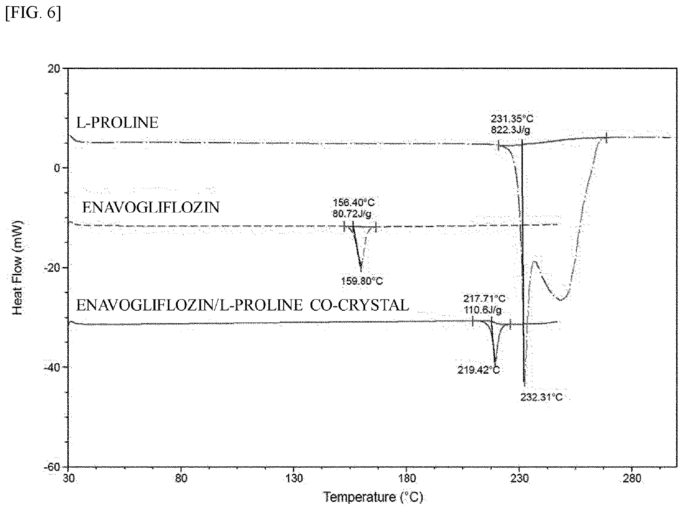

DSC was performed on the crystalline form A of enavogliflozin prepared according to Preparation Example 1 and the enavogliflozin/proline co-crystal obtained according to Preparation Example 2 using DSC Q20 obtained from TA Corp. at a scanning rate of 10 °C from 30 to 300 °C in a nitrogen gas. FIG. 5 shows the results of DSC curve of the enavogliflozin/proline co-crystal prepared in Preparation Example 2. In the differential scanning calorimetry (DSC) of the enavogliflozin/proline co-crystal, it was confirmed that the enavogliflozin/proline co-crystal exhibited an endothermic peak with an endothermic onset temperature of 217.71 °C ± 3 °C and an endothermic peak temperature of 219.42 °C ± 3 °C. FIG. 6 shows the result of comparing the DSC curves of the enavogliflozin/proline co-crystal, proline, and the crystalline form of enavogliflozin, prepared according to the examples. This shows that the crystalline form A of enavogliflozin prepared according to Preparation Example 1 and the enavogliflozin/proline co-crystal obtained according to Preparation Example 2 show distinctly different endothermic peaks at their endothermic onset temperatures and endothermic peak temperatures.

### [Experimental Example 4] Evaluation of polymorph of enavogliflozin/L-proline co-crystal

1 mL of each solvent in the following table was added to 10 mg of each of the enavogliflozin/L-proline co-crystals, the resulting solution was left for 24 hours in suspended and dissolved states, and then whether the polymorph of the enavogliflozin/L-proline co-crystal is produced was evaluated.

**[Table 4]**

| Solvent | Polarity of solvent (*p*) | DSC (endothermic reaction onset temperature, °C) | Polymorph |
|---|---|---|---|
| Water | 10.2 | 154.0 | Deformation of crystalline form A |
| Acetonitrile | 5.8 | 217.0 | Proline co-crystal |
| Ethanol | 5.2 | 214.1 | Proline co-crystal |
| Methanol | 5.1 | 217.3 | Proline co-crystal |
| Acetone | 5.1 | 209.7 | Proline co-crystal |
| Ethyl acetate | 4.4 | 215.1 | Proline co-crystal |
| Tetrahydrofuran | 4.0 | 212.3 | Proline co-crystal |
| Isopropyl alcohol | 3.9 | 215.6 | Proline co-crystal |
| Dichloromethane | 3.1 | 215.2 | Proline co-crystal |
| Diethyl ether | 2.89 | 215.4 | Proline co-crystal |
| Toluene | 2.4 | 215.8 | Proline co-crystal |
| n-Hexane | 0.1 | 215.3 | Proline co-crystal |

As a result of DSC evaluation for the crystals obtained by the above-described experiment, when water was used, the deformation of crystalline form A was obtained, but when other solvents were used, all crystals were identified as enavogliflozin/L-proline co-crystals with an endothermic onset temperature of approximately 217.71 °C ± 3 °C, and other polymorphs were not found.

### [Experimental Example 5] Evaluation of solubility of enavogliflozin/proline co-crystal

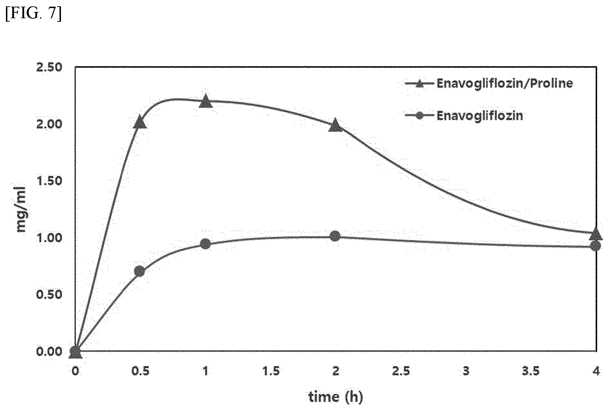

Since enavogliflozin is poorly soluble with a water solubility of 0.25 mg/mL, a proline co-crystal was prepared to improve the water solubility and gastrointestinal solubility of enavogliflozin. Through the measurement of solubility in artificial gastric fluid and artificial intestinal fluid containing a large amount of materials that maintain a dissolved state, it was confirmed that the solubility of enavogliflozin/proline co-crystal was improved compared to the crystalline form of enavogliflozin. The results are summarized in Tables 5 and 6, and FIG. 7.

**[Table 5]**

| Solubility in artificial gastric fluid (FaSSGF) | | | | | |
|---|---|---|---|---|---|
| I | Initial | 30 minutes | 1 hour | 2 hours 4 | 4 hours |
| Crystalline form A of enavogliflozin | 0.00 mg/mL | 0.31 mg/mL | 0.34 mg/mL | 0.35 mg/mL | 0.35 mg/mL |
| Enavogliflozin/proline co-crystal | 0.00 mg/mL | 0.40 mg/mL | 0.41 mg/mL | 0.43 mg/mL | 0.39 mg/mL |

**[Table 6]**

| Solubility in artificial intestinal fluid (FaSSIF) | | | | | |
|---|---|---|---|---|---|
| | Initial | 30 minutes | 1 hour | 2 hours | 4 hours |
| Crystalline form of enavogliflozin | 0.00 mg/mL | 0.69 mg/mL | 0.94 mg/mL | 1.00 mg/mL | 0.92 mg/mL |
| Enavogliflozin/proline co-crystal | 0.00 mg/mL | 2.02 mg/mL | 2.20 mg/mL | 1.98 mg/mL | 1.04 mg/mL |

As shown in Tables 5 and 6, compared to the crystalline form of enavogliflozin, the solubility of the enavogliflozin/proline co-crystal of the present invention in artificial gastric fluid and artificial intestinal fluid increased, and particularly, as shown in Table 6 and FIG. 7, it was confirmed that the solubility in the artificial intestinal fluid increased by approximately 2-fold or greater for up to 2 hours and that the solubility remained high for up to 4 hours. Accordingly, the enavogliflozin/proline co-crystal of the present invention is a novel solid form that can overcome low water solubility, which is a problem of the crystalline form of enavogliflozin, and it was predicted that the 2-fold improvement from the initial solubility in the artificial intestinal fluid can significantly improve oral absorption and maximize efficacy. The improved solubility of enavogliflozin may be particularly advantageous in the development of oral formulations for indications requiring high-dose administration.

### [Experimental Example 6] Comparison in stress stability of enavogliflozin co-crystal and crystalline form of enavogliflozin

To confirm the possibility of commercializing the enavogliflozin/proline co-crystal of the present invention (Preparation Example 2), the thermal stability of the crystalline form A of enavogliflozin (Preparation Example 1) as a control was tested at 60 °C for 4 weeks, and then the result was analyzed by high-performance liquid chromatography (HPLC). The results are shown in Table 7.

As shown in Table 7, it was confirmed that the enavogliflozin/proline co-crystal and the crystalline form of enavogliflozin remained stable without the effect of purity.

Therefore, the thermal stability of the enavogliflozin/proline co-crystal was confirmed to be equivalent to that of the crystalline form of enavogliflozin.

**[Table 7]**

| Result of stress stab ility evaluation, 60 °C, 4 weeks | | | |
|---|---|---|---|
| | Initial | 2 weeks | 4 weeks |
| Enavogliflozin/proline co-crystal | 99.9% | 99.9% | 99.8% |
| Crystalline form of enavogliflozin | 99.1% | 98.8% | 99.1% |

### [Experimental Example 7] Pharmacokinetic evaluation for enavogliflozin/proline co-crystal in beagle dogs

A test was conducted to confirm whether the pharmacokinetic profiles of enavogliflozin/proline co-crystals were improved in beagle dogs. Each of the crystalline form A of enavogliflozin and the proline co-crystal was orally administered at a dose of 1 mg/kg to male beagle dogs by setting the crystalline form A of enavogliflozin as a control and the proline co-crystal as a comparative group, and blood collection was conducted before administration (0 hour), and for 0.083, 0.25, 0.5, 0.75, 1, 1.5, 2, 4, 6, 8, 12, and 24 hours (a total of 13 times). Blood drug concentrations were analyzed by LC-MS/MS using plasma separated from the collected samples, and pharmacokinetic parameters such as the maximum observed plasma concentration (Cmax), an area under the plasma concentration-time curve (AUCₗₐₛₜ and AUC_{inf}), a time-to-maximum observed plasma concentration (Tmax), and a half-life (T_{1/2}) were calculated and interpreted using the WinNonlin 5.0.1 program.

The results are summarized in Table 8 below.

**[Table 8]**

| Pharmacokinetic evaluation for enavogliflozin/proline co-crystal in beagle dogs | | |
|---|---|---|
| Pharmacokinetic parameter | Crystalline form of enavogliflozin | Proline co-crystal |
| Tmax (hr) | 4.00 | 5.20 |
| T_{1/2} (hr) | 5.32 | 6.10 |
| Cmax (ng/mL)) | 482.20 | 482.60 |
| AUCₗₐₛₜ (ng*hr/mL) | 4340.69 | 4428.20 |
| AUC_{inf} (ng*hr/mL) | 4639.38 | 5341.58 |

As shown in Table 8, compared to the crystalline form A of enavogliflozin, the proline co-crystal of the present invention exhibited a longer the time-to-maximum observed plasma concentration (Tmax) and the half-life (T_{1/2}), along with PK exposure parameters such as AUCₗₐₛₜ and AUC_{inf}. Therefore, due to its longer half-life and higher exposure(AUC_{inf}) compared to the existing crystalline form A, the proline co-crystal remains slightly longer in the body, with its efficacy persisting for an extended duration. These pharmacokinetic characteristics of the proline co-crystal, relative to the crystalline form A, contribute to prolonged efficacy, thereby consuming energy to reduce blood glucose and potentially enhancing metabolism through secondary effects. Therefore, these pharmacokinetic properties may be useful in the development of various indications and/or formulations.

## Claims

1. An enavogliflozin/proline co-crystal which is **characterized by** an X-ray powder diffraction pattern having four or more diffraction peaks at 2[θ] values selected from 4.72 ± 0.2, 6.81 ± 0.2, 7.93 ± 0.2, 8.59 ± 0.2, 14.75 ± 0.2, 15.21 ± 0.2, 17.23 ± 0.2, 18.80 ± 0.2, 21.19 ± 0.2, 24.42 ± 0.2, and 27.29 ± 0.2.

2. The enavogliflozin/proline co-crystal of claim 1, wherein the X-ray powder diffraction pattern has a diffraction peak at 2[θ] value selected from 6.81 ± 0.2, 8.59 ± 0.2, 14.75 ± 0.2, 17.23 ± 0.2, and 18.80 ± 0.2.

3. The enavogliflozin/proline co-crystal of claim 1, which is **characterized by** an X-ray powder diffraction pattern with peak positions matching those listed in the following table:
| 2θ (±0.2°) | d (Å) | I/Iₒ(%) |
|---|---|---|
| 4.72 | 18.7 | 33.3 |
| 6.81 | 13.0 | 100.0 |
| 7.93 | 11.1 | 47.0 |
| 8.59 | 10.3 | 63.1 |
| 14.75 | 6.0 | 81.6 |
| 15.21 | 5.8 | 31.0 |
| 17.23 | 5.1 | 50.9 |
| 18.80 | 4.7 | 63.2 |
| 21.19 | 4.2 | 22.5 |
| 24.42 | 3.6 | 28.3 |
| 27.29 | 3.3 | 25.4 |

4. The enavogliflozin/proline co-crystal of claim 1, which shows an endothermic peak with an endothermic onset temperature of 217.71 °C ± 3 °C and an endothermic peak temperature of 219.42 °C ± 3 °C in differential scanning calorimetry (DSC).

5. The enavogliflozin/proline co-crystal of claim 1, wherein the enavogliflozin/proline co-crystal is in a form where 1 equivalent of proline is bound to 1 equivalent of enavogliflozin.

6. A method of preparing the enavogliflozin/proline co-crystal of any one of claims 1 to 5, comprising:
(a) mixing enavogliflozin with an organic solvent and adding proline to the resulting mixture; (b) stirring the resulting product of step (a); and (c) vacuum-drying the resulting product of step (b) to obtain enavogliflozin/proline co-crystal.

7. The method of claim 6, wherein the organic solvent is one or more types of organic solvents selected from methanol, ethanol, isopropyl alcohol, 1-butanol, acetone, tetrahydrofuran, acetonitrile, ethyl acetate, dichloromethane, methyl t-butyl ether (MTBE), toluene, and dioxane.

8. The method of claim 6, wherein the organic solvent is used in a volume 10 to 200 times the weight of enavogliflozin.

9. The method of claim 6, wherein the organic solvent is ethanol and used in a volume 10 to 60 times the weight of enavogliflozin.

10. A pharmaceutical composition comprising the enavogliflozin/proline co-crystal of any one of claims 1 to 5 as an active ingredient, and a pharmaceutically acceptable carrier.

11. The composition of claim 10, wherein the pharmaceutical composition is for treating or preventing diabetes.
